# EUROPEAN PATENT SPECIFICATION

(11) **EP 0 403 591 B1**
(45) Date of publication and mention of the grant of the patent: **14.09.1994**
(21) Application number: 89907609.5
(22) Date of filing: 20.06.1989
(51) Int. Cl.: C07K 7/38, A61K 37/02, A61K 37/40

(54) **CRF ANALOGS**
CRF-ANALOGE
ANALOGUES DE CRF

(30) Priority: 21.06.1988 US 209537
(43) Date of publication of application: 27.12.1990
(73) Proprietor: THE SALK INSTITUTE FOR BIOLOGICAL STUDIES, La Jolla California 92037 (US)
(72) Inventor: RIVIER, Jean, Edouard, Frederic, La Jolla, CA 92037 (US); VALE, Wylie, Walker, Jr., La Jolla, CA 92037 (US)
(74) Representative: Lawrence, Malcolm Graham
(86) International application number: US8902695
(87) International publication number: WO8912646

(56) References cited:
- EP-A- 0 153 845
- US-A- 4 594 329
- US-A- 4 605 642

## Description

This invention is directed to peptides and to methods for pharmaceutical treatment of mammals using such peptides. More specifically, the invention relates to analogs of the hentetracontapeptide CRF, to pharmaceutical compositions containing such CRF analogs and to methods of treatment of mammals using such CRF analogs.

### BACKGROUND OF THE INVENTION

Experimental and clinical observations have supported the concept that the hypothalamus plays a key role in the regulation of adenohypophysial corticotropic cells secretory functions. Over 25 years ago, Guillemin, Rosenberg and Saffran and Schally independently demonstrated the presence of factors in hypothalamus which would increase the rate of ACTH secretion by the pituitary gland incubated in vitro or maintained in an organ culture. None of the secretagogs characterized met the criteria expected of a physiologic corticotropin releasing factor (CRF) until ovine CRF (oCRF) was characterized in 1981 and, as disclosed in U.S. Patent No. 4,415,558, was found to have the formula:

H-Ser-Gln-Glu-Pro-Pro-Ile-Ser-Leu-Asp-Leu-Thr-Phe-His-Leu-
Leu-Arg-Glu-Val-Leu-Glu-Met-Thr-Lys-Ala-Asp-Gln-Leu-Ala-
Gln-Gln-Ala-His-Ser-Asn-Arg-Lys-Leu-Leu-Asp-Ile-Ala-NH2.

Sauvagine is a 40-residue, amidated generally similar peptide which was isolated from the skin of the South American frog Phyllomedusa sauvagei. It was characterized by Erspamer et al. and was described in Regulatory Peptides, Vol. 2 (1981), pp. 1-13. Sauvagine has the formula: 
pGlu-Gly-Pro-Pro-Ile-Ser-Ile-Asp-Leu-
Ser-Leu-Glu-Leu-Leu-Arg-Lys-Met-Ile-Glu-Ile-Glu-Lys-Gln-
Glu-Lys-Glu-Lys-Gln-Gln-Ala-Ala-Asn-Asn-Arg-Leu-Leu-Leu-
Asp-Thr-Ile-NH2.
Sauvagine and oCRF have been reported to have biological activity in lowering blood pressure in mammals and in stimulating the secretion of ACTH and β-endorphin.

Rat CRF(rCRF) has been isolated, purified and characterized as a hentetracontapeptide having the formula: 
H-Ser-Glu-Glu-Pro-Pro-Ile-Ser-Leu-Asp-Leu-
Thr-Phe-His-Leu-Leu-Arg-Glu-Val-Leu-Glu-Met-Ala-Arg-Ala-
Glu-Gln-Leu-Ala-Gln-Gln-Ala-His-Ser-Asn-Arg-Lys-Leu-Met-
Glu-Ile-Ile-NH2.
It may alternatively be referred to as rat Amunine. The formula of human CRF has now been determined to be the same as that of rCRF. Synthetic rCRF and oCRF stimulate ACTH and β-endorphin activities in vitro and in vivo and substantially lower blood pressure for an extended time period.

Analogs of these 41-residue CRF peptides of the following formula have at least substantially the same biological activity in the foregoing respects as the native peptides: 
Y-R1-R2-R3-R4-R5-Ile-Ser-R8-R9-
leu-R11-R12-R13-R14-leu-Arg-R17-R18-R19-R20-R21-R22-
R23-R24-R25-R26-R27-R28-R29-Gln-ala-R32-R33-Asn-Arg-
R36-R37-R38-R39-R40-R41-NH2
wherein Y is an acyl group having 7 or less carbon atoms or hydrogen; R₁ is Ser, D-Ser or des R₁; R₂ is Glu, Gln, pGlu, D-pGlu or des R₂; R₃ is Glu, Gly, D-Tyr or des R₃; R₄ is Pro, D-Pro or des R₄; R₅ is Pro or desR₅; R₈ and R₁₉ are selected from the group consisting of leu, Ile, ala, Gly, Val, Nle, Phe and Gln; R₉ is Asp or Glu; R₁₁ is Thr or Ser; R₁₂ is Phe, leu, ala, Ile, Gly, Val, Nle or Gln; R₁₃ is His, Tyr or Glu; R₁₄ is leu or Met; R₁₇ is Glu or Lys; R₁₈ is Val, Nle or Met; R₂₀ is His; R₂₁ is Arg, Met, Nva, Ile, ala, leu, Nle, Val, Phe or Gln; R₂₂ is ala, Thr, Asp or Glu; R₂₃ is Arg, Orn, Har or Lys; R₂₄ is Met, leu, Ile, ala, Gly, Val, Nle, Phe and Gln; R₂₅ is Glu or Asp; R₂₆ is Gly, Gln, Asn or Lys; R₂₇ is leu, Ile, ala, Val, Nva, Met, Nle, Phe, Asp, Asn, Gln or Glu; R₂₈ is ala, Arg or Lys; R₂₉ is Gln or Glu; R₃₂ is Leu, His, Gly, Tyr or ala; R₃₃ is Ile, Ser, Asn, leu, Thr or ala; R₃₆ is Asn, Lys, Orn, Arg, Har or Leu; R₃₇ is leu or Tyr; R₃₈ is Met or leu; R₃₉ is Glu or Asp; R₄₀ is Ile, Thr, Glu, ala, Val, leu, Nle Phe, Nva, Gly, Asn or Gln; and R₄₁ is Ile, ala, Gly, Val, Leu, Nle, Phe, Gln or des R₄₁. Nontoxic addition salts thereof are also included within the scope of the invention.

Pharmaceutical compositions in accordance with the invention include such CRF analogs, or nontoxic addition salts thereof, dispersed in a pharmaceutically or veterinarily acceptable liquid or solid carrier. The administration of such peptides or pharmaceutically or veterinarily acceptable addition salts thereof to mammals, particularly humans, in accordance with the invention may be carried out for the regulation of secretion of ACTH, β-endorphin, β-lipotropin, other products of the pro-opiomelanocortin gene and corticosterone and/or for the lowering of blood pressure and/or for affecting mood, behavioral and gastrointestinal functions and autonomic nervous system activities. Furthermore CRF analogs may be used for the evaluation of the status of pituitary, cardiovascular, gastrointestinal or central nervous system functions.

The nomenclature used to define the peptides is that specified by Schroder & Lubke, "The Peptides", Academic Press (1965) wherein, in accordance with conventional representation, the amino group appears to the left and the carboxyl group to the right. The standard 3-letter abbreviations to identify the alpha-amino acid residues, and where the amino acid residue has isomeric forms, it is the L-form of the amino acid that is represented unless otherwise expressly indicated, e.g. Ser = L-serine, Nle = L-norleucine, Nva = norvaline, Har = homoarginine, Orn = ornithine etc. In addition the following abbreviations are used: leu = either L-leucine or C CH₃-L-leucine (CML) and ala = either L-alanine or C CH₃-L-alanine (CMA).

The invention provides analogs of CRF of the following Formula (I): 
Y-R1-R2-R3-R4-R5-Ile-Ser-
R8-R9-leu-R11-R12-R13-R14-leu-Arg-R17-R18-R19-R20-
R21-R22-R23-R24-R25-R26-R27-R28-R29-Gln-ala-R32-
R33-Asn-Arg-R36-R37-R38-R39-R40-R41-NH2
wherein Y is an acyl group having 7 or less carbon atoms or hydrogen; R₁ is Ser, D-Ser or des R₁; R₂ is Glu, Gln, pGlu, D-pGlu or des R₂; R₃ is Glu, Gly, D-Tyr or des R₃; R₄ is Pro, D-Pro or des R₄; R₅ is Pro or desR₅; R₈ and R₁₉ are selected from the group consisting of leu, Ile, ala, Gly, Val, Nle, Phe and Gln; R₉ is Asp or Glu; R₁₁ is Thr or Ser; R₁₂ is Phe, leu, ala, Ile, Gly, Val, Nle or Gln; R₁₃ is His, Tyr or Glu; R₁₄ is leu or Met; R₁₇ is Glu or Lys; R₁₈ is Val, Nle or Met; R₂₀ is His; R₂₁ is Arg, Met, Nva, Ile, ala, leu, Nle, Val, Phe or Gln; R₂₂ is ala, Thr, Asp or Glu; R₂₃ is Arg, Orn, Har or Lys; R₂₄ is Met, leu, Ile, ala, Gly, Val, Nle, Phe and Gln; R₂₅ is Glu or Asp; R₂₆ is Gly, Gln, Asn or Lys; R₂₇ is leu, Ile, ala, Val, Nva, Met, Nle, Phe, Asp, Asn, Gln or Glu; R₂₈ is ala, Arg or Lys; R₂₉ is Gln or Glu; R₃₂ is Leu, His, Gly, Tyr or ala; R₃₃ is Ile, Ser, Asn, leu, Thr or ala; R₃₆ is Asn, Lys, Orn, Arg, Har or Leu; R₃₇ is leu or Tyr; R₃₈ is Met or leu; R₃₉ is Glu or Asp; R₄₀ is Ile, Thr, Glu, ala, Val, leu, Nle, Phe, Nva, Gly, Asn or Gln; R₄₁ is Ile, ala, Gly, Val, Leu, Nle, Phe, Gln or des R₄₁. Nontoxic addition salts of these peptides can be used as well. In this formula, preferably at least one of the following substituents are present: R₂₁ is Arg, R₂₆ is Gly, and/or R₃₆ is Asn.

These analogs are considered to be at least as potent as native CRF. These analogs may include residues having a high alpha-helical forming potential such as: R₁ is Ser, R₂ is Gln or Glu, R₃ is Glu, R₄ and R₅ are Pro, R₈ is leu, R₁₁ is Thr, R₁₂ is Phe or leu, R₁₃ is His or Glu, R₁₇ is Glu, R₁₈ and R₂₁ are Met or Nle, R₁₉ and R₃₇ are leu, R₂₂ and R₄₁ are ala, R₂₃ is Lys, R₂₄ and R₂₈ are ala, R₂₅ and R₃₉ are Glu, R₂₆ is Gln, R₂₇ is Glu or leu, R₂₉ is Glu, R₃₂ is His or ala, R₃₃ is Ser or leu, R₃₈ is Leu and R₄₀ is Ile or Glu. One analog which has been found to be particularly potent is: 
H-Ser-Gln-Glu-
Pro-Pro-Ile-Ser-Leu-Asp-Leu-Thr-Phe-His-Leu-Leu-Arg-Glu-
Met-Leu-His-Met-Ala-Lys-Ala-Glu-Gln-Glu-Ala-Glu-Gln-Ala-
Ala-Leu-Asn-Arg-Leu-Leu-Leu-Glu-Glu-Ala-NH2.
It remains potent even if slightly shortened at the N-terminus, i.e., by a sequence of up to about 5 residues.

Two other analogs which are particularly potent are those having the formulas:

H-Ser-Gln-Glu-Pro-Pro-
Ile-Ser-Leu-Asp-Leu-Thr-Phe-His-Leu-Leu-Arg-Glu-Met-Leu-
His-Met-Ala-Lys-Ala-Glu-Gln-Glu-Ala-Glu-Gln-Ala-Ala-Leu-
Asn-Arg-Asn-Leu-Leu-Glu-Glu-Ala-NH2 and H-Ser-Gln-Glu-
Pro-Pro-Ile-Ser-Leu-Asp-Leu-Thr-Phe-His-Leu-Leu-Arg-Glu-
Met-Leu-His-Met-Ala-Lys-Ala-Glu-Gly-Glu-Ala-Glu-Gln-Ala-
Ala-Leu-Asn-Arg-Leu-Leu-Leu-Glu-Glu-Ala-NH2.

The peptides are synthesized by a suitable method, such as by exclusively solid-phase techniques, by partial solid-phase techniques, by fragment condensation or by classical solution addition. Certain CRF analogs which do not include D-isomer residues or unnatural amino acid residues may also be synthesized by recently developed recombinant DNA techniques.

Synthesis by the use of recombinant DNA techniques, for purposes of this application, should be understood to include the suitable employment of a structural gene coding for the desired form of CRF analog. Such a synthetic CRF peptide may be obtained by transforming a microorganism using an expression vector including a promoter and operator together with such structural gene and causing such transformed microorganism to express the CRF analog peptide. A non-human animal may also be used to produce the CRF analog peptide by gene-farming, the general techniques of which are known to those skilled in the art. For example, microinjection of embryos as described in W083/01783, published 26 May 1983, and W082/04443, published 23 December 1982, may be used. The synthetic CRF analog peptide is then suitably recovered from the animal by extraction from sera or the like.

Common to chemical syntheses of peptides is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with various of these residues having side-chain protecting groups.

Thus, chemical synthesis of such a peptide analog may result in the formation of an intermediate of the Formula (II): 
X^1-R_1(X^2)-R_2(X^4 or X^5)-R_3(X^5 or X)-R_4-R_5-Ile-Ser(X^2)-
R_8-R_9(X^5)-leu-R_11(X^2)-R_12(X^4)-R_13(X or X^5)-R_14-leu-
Arg(X^3)-R_17(X^5 or X^6)-R_18-R_19(X^4)-R_20(X)-
R_21(X^3)-R_22(X^2 or X^5)-R_23(X^3 or X^6)-R_24(X^4)-R_25(X^5)-
R_26(X^4 or X^6)-R_27(X^4 or X^5)-R_28(X^3 or X^6)-R_29(X^4 or
X^5)-Gln(X^4)-ala-R_32(X)-R_33(X^2 or X^4)-Asn(X^4)-Arg(X^3)-
 
R36(X6 or X4)-R37(X)-R38-R39(X5)-R40(X2 or X4 or X5)-
R41(X4)-X7
wherein: the R-groups are as hereinbefore defined.

X¹ is either hydrogen or an α-amino protecting group. The α-amino protecting groups contemplated by X¹ are those known to be useful in the art in the step-wise synthesis of polypeptides. Among the classes of -amino protecting groups covered by X¹ are (1) acyl-type protecting groups, such as formyl, acrylyl(Acr), benzoyl(Bz) and acetyl(Ac) which are preferably used only at the N-terminal; (2) aromatic urethan-type protecting groups, such as benzyloxycarbonyl(Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (BOC), diisopropylmethoxycarbonyl, isopropyloxycarbonyl, ethoxycarbonyl, allyloxycarbonyl; (4) cycloalkyl urethan-type protecting groups, such as fluorenylmethyloxycarbonyl(FMOC), cyclopentyloxycarbonyl, adamantyloxycarbonyl,and cyclohexyloxycarbonyl; and (5) thiourethan-type protecting groups, such as phenylthiocarbonyl. The preferred α-amino protecting group is BOC.

X² is a protecting group for the hydroxyl group of Thr and Ser and is preferably selected from the class consisting of acetyl(Ac), benzoyl(Bz), tert-butyl, triphenylmethyl(trityl), tetrahydropyranyl, benzyl ether(Bzl) and 2,6-dichlorobenzyl (DCB). The most preferred protecting group is Bzl. X² can be hydrogen, which means there is no protecting group on the hydroxyl group.

X³ is a protecting group for the guanidino group of Arg or Har preferably selected from the class consisting of nitro, p-toluenesulfonyl(Tos), Z, adamantyloxycarbonyl and BOC, or is hydrogen. Tos is most preferred.

X⁴ is hydrogen or a protecting group, preferably xanthyl(Xan), for the amido group of Asn or Gln.

X⁵ is hydrogen or an ester-forming protecting group for the β- or γ-carboxyl group of Asp or Glu, preferably selected from the class consisting of benzyl, 2,6-dichlorobenzyl, methyl, ethyl and t-butyl ester. OBzl is most preferred.

X⁶ is hydrogen or a protecting group for the side chain amino substituent of Lys or Orn. Illustrative of suitable side chain amino protecting groups are Z, 2-chlorobenzyloxycarbonyl(2-Cl-Z), Tos, t-amyloxycarbonyl(Aoc), BOC and aromatic or aliphatic urethan-type protecting groups as specified hereinbefore.

When His is present, X is hydrogen or a protecting group for the imidazole nitrogen such as Tos or 2,4-dinitrophenyl(DNP), and when Tyr is present, X is hydrogen or a protecting group for the hydroxyl group such as DCB. When Met is present, the sulfur may be protected, if desired, with oxygen.

The selection of a side chain amino protecting group is not critical except that it should must be one which is not removed during deprotection of the α-amino groups during the synthesis. Hence, the α-amino protecting group and the side chain amino protecting group cannot be the same.

X⁷ is NH₂, a protecting group such as an ester or an anchoring bond used in solid phase synthesis for linking to a solid resin support, preferably one represented by the formulae:

-NH-benzhydrylamine (BHA) resin support and

-NH-paramethylbenzhydrylamine (MBHA) resin support.

Cleavage from a BHA or MBHA resin directly gives the CRF analog amide. By employing a methyl-derivative of such a resin, a methyl-substituted amide can be created which is considered to be the equivalent of the unsubstituted amide.

In the formula for the intermediate, at least one of X, X¹, X², X³, X⁴, X⁵ and X⁶ is a protecting group. The particular amino acid chosen for each the R-group determines whether there will also be a protecting group attached as specified hereinbefore and as generally known in the art. In selecting a particular side chain protecting group to be used in the synthesis of the peptides, the following rules are followed: (a) the protecting group should be stable to the reagent and under the reaction conditions selected for removing the α-amino protecting group at each step of the synthesis, (b) the protecting group should retain its protecting properties and not be split off under coupling conditions and (c) the side chain protecting group must be removable, upon the completion of the synthesis containing the desired amino acid sequence, under reaction conditions that will not alter the peptide chain.

For the acyl group at the N-terminus represented by Y, acetyl, formyl, acrylyl and benzoyl are preferred. For the 1 to 10 amino acid peptide which may be optionally included without adversely affecting the potency, any amino acids may be used, but the L- or D-forms of the naturally occurring amino acids would normally be used. Moreover, as indicated hereinbefore, the N-terminus can be slightly shortened without significantly affecting biological potency.

Thus, the present invention is also considered to provide a process for the manufacture of compounds defined by the Formula (I) comprising (a) forming a peptide having at least one protective group and having the Formula (II) wherein: X, X¹, X², X³, X⁴, X⁵ and X⁶ are each either hydrogen or a protective group, and X⁷ is either a protective group or an anchoring bond to resin support or NH₂ and (b) splitting off the protective group or groups or anchoring bond from said peptide of the Formula (II) and (c) if desired, converting a resulting peptide into a nontoxic addition salt thereof.

When the peptides are prepared by chemical synthesis, they are preferably prepared using solid phase synthesis, such as that described by Merrifield, J. Am. Chem. Soc., 85, p 2149 (1964), although other equivalent chemical syntheses known in the art can also be used as previously mentioned. Solid-phase synthesis is usually commenced from the C-terminus end of the peptide by coupling a protected α-amino acid to a suitable resin as generally set forth in U.S. Patent No. 4,244,946 issued Jan. 21, 1981 to Rivier et al., the disclosure of which is incorporated herein by reference. Such a starting material for rCRF analogs can be prepared by attaching α-amino-protected Ile to a BHA resin.

Ile protected by BOC is coupled to the BHA resin using methylene chloride and dimethylformamide (DMF). Following the coupling of BOC-Ile to the resin support, the α-amino protecting group is removed, as by using trifluoroacetic acid(TFA) in methylene chloride, TFA alone or with HCl in dioxane. Preferably 50 volume % TFA in methylene chloride is used with 0-5 weight % 1,2 ethanedithiol. The deprotection is carried out at a temperature between about 0C and room temperature. Other standard cleaving reagents and conditions for removal of specific α-amino protecting groups may be used as described in Schroder & Lubke, "The Peptides" 1, pp 72-75 (Academic Press 1965).

After removal of the α-amino protecting group of Ile, the remaining α-amino- and side chain-protected amino acids are coupled step-wise in the desired order to obtain the intermediate compound defined hereinbefore. As an alternative to adding each amino acid separately in the synthesis, some of them may be coupled to one another prior to addition to the solid phase reactor. The selection of an appropriate coupling reagent is within the skill of the art. Particularly suitable as coupling reagents are N,N'-dicyclohexyl carbodiimide(DCCI) and N,N'-diisopropyl carbodiimide(DICI).

The activating reagents used in the solid phase synthesis of the peptides are well known in the peptide art. Examples of suitable activating reagents are carbodiimides, such as N,N'-diisopropyl carbodiimide and N-ethyl-N'-(3-dimethylaminopropyl)carbodiimide. Other activating reagents and their use in peptide coupling are described by Schroder & Lubke, supra, in Chapter III and by Kapoor, J. Phar. Sci., 59, pp 1-27 (1970).

Each protected amino acid or amino acid sequence is introduced into the solid phase reactor in about a fourfold excess, and the coupling is carried out in a medium of dimethylformamide(DMF):CH₂Cl₂ (1:1) or in DMF or CH₂Cl₂ alone. In instances where the coupling is carried out manually, the success of the coupling reaction at each stage of the synthesis is monitored by the ninhydrin reaction, as described by E. Kaiser et al., Anal. Biochem. 34, 595 (1970). In cases where incomplete coupling occurs, the coupling procedure is repeated before removal of the α-amino protecting group prior to the coupling of the next amino acid. The coupling reactions can be performed automatically, as on a Beckman 990 automatic synthesizer, using a program such as that reported in Rivier et al., Biopolymers, 1978, 17, pp.1927-1938.

After the desired amino acid sequence has been completed, the intermediate peptide is removed from the resin support by treatment with a reagent, such as liquid hydrogen fluoride, which not only cleaves the peptide from the resin but also cleaves all remaining side chain protecting groups X, X², X³, X⁴, X⁵ and X⁶ and the α-amino protecting group X¹ (unless it is an acyl group which is intended to be present in the final peptide), to obtain the peptide. When using hydrogen fluoride for cleaving, anisole or cresole and methylethyl sulfide are included in the reaction vessel as scavengers. When Met is present in the sequence, the BOC protecting group may be cleaved with trifluoroacetic acid(TFA)/ethanedithiol prior to cleaving the peptide from the resin to eliminate potential S-alkylation.

The following Example sets forth the preferred method for synthesizing CRF analogs by the solid-phase technique.

### EXAMPLE I

The synthesis of [His²⁰]-rCRF having the formula: 
H-Ser-Glu-Glu-Pro-Pro-Ile-Ser-Leu-Asp-Leu-Thr-
Phe-His-Leu-Leu-Arg-Glu-Val-Leu-His-Met-Ala-Arg-Ala-Glu-
Gln-Leu-Ala-Gln-Gln-Ala-His-Ser-Asn-Arg-Lys-Leu-Met-Glu-
Ile-Ile-NH2
is conducted in a stepwise manner on a MBHA hydrochloride resin, such as available from Bachem, Inc., having a substitution range of about 0.1 to 0.5 mmoles/gm. resin. The synthesis is performed on an automatic Beckman 990B peptide synthesizer using a suitable program, preferably as follows:

| STEP | REAGENTS AND OPERATIONS | MIX TIMES MIN. |
|---|---|---|
| 1 | CH₂Cl₂ wash-80 ml. (2 times) | 3 |
| 2 | Methanol(MeOH) wash-30 ml. (2 times) | 3 |
| 3 | CH₂Cl₂ wash-80 ml. (3 times) | 3 |
| 4 | 50 percent TFA plus 5 percent 1,2-ethanedithiol in CH₂Cl₂-70 ml. (2 times) | 12 |
| 5 | Isopropanol wash-80 ml. (2 times) | 3 |
| 6 | TEA 12.5 percent in CH₂Cl₂-70 ml. (2 times) | 5 |
| 7 | MeOH wash-40 ml. (2 times) | 2 |
| 8 | CH₂Cl₂ wash-80 ml. (3 times) | 3 |
| 9 | Boc-amino acid (10 mmoles) in 30 ml. of either DMF or CH₂Cl₂, depending upon the solubility of the particular protected amino acid, (1 time) | |
| | plus DCCI (10 mmoles) in CH₂Cl₂ | 30-300 |

Coupling of BOC-Ile results in the substitution of about 0.35 mmol. Ile per gram of resin. All solvents that are used are carefully degassed, preferably by sparging with an inert gas, e.g. helium or nitrogen, to insure the absence of oxygen that might undesirably oxidize the sulfur of the Met residue.

After deprotection and neutralization, the peptide chain is built step-by-step on the resin. Generally, one to two mmol. of BOC-protected amino acid in methylene chloride is used per gram of resin, plus one equivalent of 2 molar DCCI in methylene chloride, for two hours. When BOC-Arg(Tos) is being coupled, a mixture of 50% DMF and methylene chloride is used. Bzl is used as the hydroxyl side-chain protecting group for Ser and Thr. P-nitrophenyl ester(ONp) is used to activate the carboxyl end of Asn or Gln, and for example, BOC-Asn(ONp) is coupled overnight using one equivalent of HOBt in a 50% mixture of DMF and methylene chloride. The amido group of Asn or Gln is protected by Xan when DCCI coupling is used instead of the active ester method. 2-Cl-Z is used as the protecting group for the Lys side chain. Tos is used to protect the guanidino group of Arg and the imidazole group of His, and the side chain carboxyl group of Glu or Asp is protected by OBzl. At the end of the synthesis, the following composition is obtained 
BOC-Ser(Bzl)-Glu(OBzl)-
Glu(OBzl)-Pro-Pro-Ile-Ser(Bzl)-Leu-Asp(OBzl)-Leu-Thr(Bzl)-
Phe-His(Tos)-Leu-Leu-Arg(Tos)-Glu(OBzl)-Val-Leu-His(TOS)-
Met-Ala-Arg(Tos)-Ala-Glu(OBzl)-Gln(Xan)-Leu-Ala-Gln(Xan)-
Gln(Xan)-Ala-His(Tos)-Ser(Bzl)-Asn(Xan)-Arg(Tos)-Lys
(2-Cl-Z)-Leu-Met-Glu(OBzl)-Ile-Ile-resin support.
Xan may have been partially or totally removed by TFA treatment used to deblock the α-amino protecting group.

In order to cleave and deprotect the resulting protected peptide-resin, it is treated with 1.5 ml. anisole, 0.5 ml. of methylethylsulfide and 15 ml. hydrogen fluoride (HF) per gram of peptide-resin, first at -20°C. for 20 min. and then at 0°C. for one-half hour. After elimination of the HF under high vacuum, the resin-peptide is washed alternately with dry diethyl ether and chloroform, and the peptides are then extracted with de-gassed 2N aqueous acetic acid and separated from the resin by filtration.

The peptide is purified by gel permeation followed by semi-preparative HPLC as described in Rivier et al., Peptides: Structure and Biological Function (1979) pp. 125-128, and Rivier et al., J. Chromatography (1983). The chromatographic fractions are carefully monitored by HPLC, and only the fractions showing substantial purity were pooled.

To check whether the precise sequence was achieved, the rCRF analog is hydrolyzed in sealed evacuated tubes containing constant boiling HCl, 3µl of thioglycol/ml. and 1 nmol of Nle (as an internal standard) for 9 hours at 140°C. Amino acid analyses of the hydrolysates using a Beckman 121 MB amino acid analyzer show the following amino acid ratios: Asx(1.8), Thr(0.9), Ser(3.1), Glx(7.9), Pro(2.2), Ala(3.9), Val(0.9), Met(1.8), Ile(2.8), Leu(6.9), Phe(0.8), Lys(1.0), His(2.9) and Arg(2.9), which confirms that the 41-residue peptide structure is obtained.

### EXAMPLE II

The synthetic peptide having the formula: 
H-Ser-Gln-Glu-Pro-Pro-Ile-Ser-Leu-Asp-Leu-Thr-Phe-His-
Leu-Leu-Arg-Glu-Met-Leu-His-Met-Ala-Lys-Ala-Glu-Gln-Glu-
Ala-Glu-Gln-Ala-Ala-Leu-Asn-Arg-Leu-Leu-Leu-Glu-Glu-Ala-
NH2
is synthesized using a procedure generally as set forth in Example I.

The peptide is purified in the manner set forth in Example I. Amino acid analysis shows the expected amino acid ratios.

### EXAMPLE III

The synthetic peptide from Example I and synthetic oCRF are examined for their effects on the secretion of ACTH and β-endorphin in vitro and also in vivo.

The potency of synthetic oCRF to stimulate the secretion of ACTH and β-endorphin by cultured rat pituitary cells is measured using the procedure as generally set forth in Endocrinology, 91, 562 (1972). Half-maximal responses are observed at concentrations of the peptide very substantially less than the synthetic oCRF concentrations of about 250 picomolar which are needed to achieve this response. In vivo testing is carried out using the general procedure set forth in C. Rivier et al., Science, 218, 377 (1982), and biological potency is ascertained.

### EXAMPLE IV

The peptide [His²⁰, Gly²⁶)-rCRF having the formula: 
H-Ser-Glu-Glu-Pro-Pro-Ile-Ser-Leu-Asp-Leu-Thr-Phe-His-Leu-
Leu-Arg-Glu-Val-Leu-His-Met-Ala-Arg-Ala-Glu-Gly-Leu-
Ala-Gln-Gln-Ala-His-Ser-Asn-Arg-Lys-Leu-Met-Glu-Ile-Ile-
NH2
is synthesized. Testing in accordance with the general procedure set forth in Example III shows that it likewise stimulates the secretion of ACTH and β-END-LI and causes a very significant lowering of blood pressure.

### EXAMPLE V

The peptide [Glu⁹, His²⁰, Nle²¹, Glu²⁹, Ile³³]-rCRF having the formula: 
H-Ser-Glu-Glu-Pro-Pro-Ile-Ser-Leu-Glu-Leu-Thr-Phe-His-Leu-
Leu-Arg-Glu-Val-Leu-His-Nle-Ala-Arg-Ala-Glu-Gln-Leu-Ala-
Glu-Gln-Ala-His-Ile-Asn-Arg-Lys-Leu-Met-Glu-Ile-Ile-NH2
is synthesized. Testing in accordance with the general procedure set forth in Example III shows that it likewise stimulates the secretion of ACTH and β-END-LI and causes a very significant lowering of blood pressure to a greater extent than rCRF.

### EXAMPLE VI

The peptide [Benzoyl-Gly¹, Nle¹², His²⁰, Arg³⁶]-rCRF having the formula: 
Bz-Gly-Glu-Glu-Pro-Pro-Ile-Ser-Leu-Asp-Leu-Thr-Nle-His-
Leu-Leu-Arg-Glu-Val-Leu-His-Met-Ala-Arg-Ala-Glu-Gln-Leu-
Ala-Gln-Gln-Ala-His-Ser-Asn-Arg-Arg-Leu-Met-Glu-Ile-Ile-
 NH2
is synthesized. Testing in accordance with the general procedure set forth in Example III shows that it likewise stimulates the secretion of ACTH and β-END-LI and causes a very significant lowering of blood pressure.

### EXAMPLE VII

The peptide [Acetyl-Pro⁴, His²⁰, Glu^{29,40}, Asn³⁶]-rCRF(4-41) having the formula: 
Ac-Pro-Pro-Ile-Ser-Leu-Asp-Leu-Thr-Phe-His-Leu-Leu-Arg-
Glu-Val-Leu-His-Met-Ala-Arg-Ala-Glu-Gln-Leu-Ala-Glu-Gln-
Ala-His-Ser-Asn-Arg-Asn-Leu-Met-Glu-Glu-Ile-NH2
is synthesized. Testing in accordance with the general procedure set forth in Example III shows that it likewise stimulates the secretion of ACTH and β-END-LI and causes a very significant lowering of blood pressure to a greater extent than rCRF.

### EXAMPLE VIII

The peptide [His²⁰, Nle²¹, Leu³², Ile³³, Glu⁴⁰]-rCRF having the formula: 
H-Ser-Glu-Glu-Pro-Pro-Ile-Ser-Leu-Asp-Leu-Thr-Phe-His-Leu-
Leu-Arg-Glu-Val-Leu-His-Nle-Ala-Arg-Ala-Glu-Gln-Leu-Ala-
Gln-Gln-Ala-Leu-Ile-Asn-Arg-Lys-Leu-Met-Glu-Glu-Ile-NH2
is synthesized. Testing in accordance with the general procedure set forth in Example III shows that it likewise stimulates the secretion of ACTH and β-END-LI and causes a very significant lowering of blood pressure to a greater extent than rCRF.

### EXAMPLE IX

The peptide [His²⁰, Arg²¹, Leu³², Ile³³]-rCRF having the formula: 
H-Ser-Glu-Glu-Pro-Pro-Ile-Ser-Leu-Asp-Leu-Thr-Phe-His-Leu-
Leu-Arg-Glu-Val-Leu-His-Arg-Ala-Arg-Ala-Glu-Gln-Leu-Ala-
Gln-Gln-Ala-Leu-Ile-Asn-Arg-Lys-Leu-Met-Glu-Ile-Ile-NH2
is synthesized. Testing in accordance with the general procedure set forth in Example III shows that it likewise stimulates the secretion of ACTH and β-END-LI and causes a very significant lowering of blood pressure to a greater extent than rCRF.

### EXAMPLE X

The peptide having the formula: 
H-Ser-Gln-Glu-Pro-Pro-Ile-Ser-Leu-Glu-Leu-Thr-Phe-His-
Leu-Leu-Arg-Glu-Nle-Leu-His-Arg-Ala-Lys-Ala-Glu-Gln-Glu-
Ala-Glu-Gln-Ala-Ala-Leu-Asn-Arg-Leu-Leu-Leu-Glu-Glu-Ala-
NH2
is synthesized. Testing in accordance with the general procedure set forth in Example III shows that it likewise stimulates the secretion of ACTH and β-END-LI and causes a very significant lowering of blood pressure.

### EXAMPLE XI

The peptide having the formula: 
H-Ser-Gln-Glu-Pro-Pro-Ile-Ser-Leu-Asp-Leu-Thr-Phe-His-
Leu-Leu-Arg-Glu-Nle-Leu-His-Nle-Ala-Lys-Ala-Glu-Gln-Glu-
Ala-Glu-Gln-Ala-Ala-Leu-Asn-Arg-Leu-Leu-Leu-Glu-Glu-Ala-
NH2
is synthesized. Testing in accordance with the general procedure set forth in Example III shows that it likewise stimulates the secretion of ACTH and β-END-LI and causes a very significant lowering of blood pressure.

### EXAMPLE XII

The peptide having the formula: 
H-Ser-Gln-D-Tyr-Pro-Pro-Ile-Ser-Leu-Asp-Leu-Thr-Phe-His-
Leu-Leu-Arg-Glu-Nle-Leu-His-Nle-Ala-Lys-Ala-Gly-Gln-Glu-
Ala-Glu-Gln-Ala-Ala-Leu-Asn-Arg-Leu-Leu-Leu-Glu-Glu-Ala-
NH2
is synthesized. Testing in accordance with the general procedure set forth in Example III shows that it likewise stimulates the secretion of ACTH and β-END-LI and causes a very significant lowering of blood pressure.

### EXAMPLE XIII

The synthetic peptide having the formula: 
H-Ser-Gln-Glu-Pro-Pro-Ile-Ser-Leu-Asp-Leu-Thr-Phe-His-
Met-Leu-Arg-Glu-Met-Leu-His-Arg-Ala-Lys-Met-Glu-Gly-Glu-
Ala-Glu-Gln-Ala-Leu-Ile-Asn-Arg-Asn-Leu-Leu-Glu-Glu-Ala-
NH2
is synthesized. Testing in accordance with the general procedure set forth in Example III shows that it likewise stimulates the secretion of ACTH and β-END-LI and causes a very significant lowering of blood pressure.

### EXAMPLE XIV

The peptide [His²⁰, Asn³⁶]-oCRF having the formula: 
H-Ser-Gln-Glu-Pro-Pro-Ile-Ser-Leu-Asp-Leu-Thr-Phe-His-Leu-
Leu-Arg-Glu-Val-Leu-His-Met-Thr-Lys-Ala-Asp-Gln-Leu-Ala-
Gln-Gln-Ala-His-Ser-Asn-Arg-Asn-Leu-Leu-Asp-Ile-Ala-NH2
is synthesized. Testing in accordance with the general procedure set forth in Example III shows that it stimulates the secretion of ACTH and β-END-LI and also causes a very significant lowering of blood pressure.

### EXAMPLE XV

The peptide [His²⁰, Nle²¹]-oCRF having the formula: 
H-Ser-Gln-Glu-Pro-Pro-Ile-Ser-Leu-Asp-Leu-Thr-Phe-His-Leu-
Leu-Arg-Glu-Val-Leu-His-Nle-Thr-Lys-Ala-Asp-Gln-Leu-Ala-
Gln-Gln-Ala-His-Ser-Asn-Arg-Lys-Leu-Leu-Asp-Ile-Ala-NH2
is synthesized. Testing in accordance with the general procedure set forth in Example III shows that it likewise stimulates the secretion of ACTH and β-END-LI and causes a very significant lowering of blood pressure.

### EXAMPLE XVI

The peptide [D-Pro⁴, His²⁰]-oCRF(4-41) having the formula: 
H-D-Pro-Ile-Ser-Leu-Asp-Leu-Thr-Phe-His-Leu-Leu-Arg-Glu-
Val-Leu-His-Met-Thr-Lys-Ala-Asp-Gln-Leu-Ala-Gln-Gln-Ala-
His-Ser-Asn-Arg-Lys-Leu-Leu-Asp-Ile-Ala-NH2
is synthesized. Testing in accordance with the general procedure set forth in Example III shows that it likewise stimulates the secretion of ACTH and β-END-LI and causes a very significant lowering of blood pressure.

### EXAMPLE XVII

The peptide having the formula: 
H-Pro-Pro-Ile-Ser-Leu-Asp-Leu-Thr-Phe-His-Leu-Leu-Arg-Glu-
Nle-Leu-His-Nle-Ala-Arg-Ala-Glu-Gln-Glu-Ala-Glu-Gln-Ala-
Leu-Leu-Asn-Arg-Asn-Leu-Leu-Glu-Glu-Ala-NH2
is synthesized. Testing in accordance with the general procedure set forth in Example III shows that it likewise stimulates the secretion of ACTH and β-END-LI and causes a very significant lowering of blood pressure.

### EXAMPLE XVIII

The synthetic peptide having the formula: 
H-Ser-Gln-Glu-Pro-Pro-Ile-Ser-Leu-Asp-Leu-Thr-Phe-His-
Met-Leu-Arg-Glu-Met-Leu-His-Met-Ala-Lys-Met-Glu-Gln-Glu-
Ala-Glu-Gln-Ala-Ala-Leu-Asn-Arg-Leu-Leu-Leu-Glu-Glu-Ala-
NH2
is synthesized using a procedure generally as set forth in Example I. Testing in accordance with the general procedure set forth in Example III shows that it likewise stimulates the secretion of ACTH and β-END-LI and causes a very significant lowering of blood pressure.

### EXAMPLE XIX

The synthetic peptide having the formula: 
H-Ser-Gln-Glu-Pro-Pro-Ile-Ser-Leu-Asp-Leu-Thr-Phe-His-
Leu-Leu-Arg-Glu-Met-Leu-His-Arg-Ala-Lys-Ala-Glu-Gln-Glu-
Ala-Glu-Gln-Ala-Ala-Ile-Asn-Arg-Leu-Leu-Leu-Glu-Glu-Ala-
NH2
is synthesized using a procedure generally as set forth in Example I. Testing in accordance with the general procedure set forth in Example III shows that it likewise stimulates the secretion of ACTH and β-END-LI and causes a very significant lowering of blood pressure.

CRF analogs exhibit such lowering of blood pressure that they may be particularly valuable for the treatment of high blood pressure conditions and also for the treatment of patients who are to undergo certain types of surgery.

CRF profoundly stimulates the pituitary-adrenalcortical axis, and CRF analogs should be useful to stimulate the functions of this axis in some types of patients with low endogenous glucocorticoid production. For example, CRF should be useful in restoring pituitary-adrenal function in patients having received exogenous glucocorticoid therapy whose pituitary-adrenalcortical functions remain suppressed

Most other regulatory peptides have been found to have effects upon the central nervous system and upon the gastrointestinal tract. Because ACTH and β-END secretion is the "sine qua non" of mammal's response to stress, it was not surprising that CRF has significant effects on the brain as a mediator of the body's stress response. Accordingly, CRF should also find application in modifying the mood, learning and behavior of normal and mentally disordered individuals. Because CRF analogs elevate the levels of ACTH, β-END, β-lipotropin, other pro-opiomelanocortin gene products and corticosterone, its administration can be used to induce their effects on the brain and its periphery to thereby influence memory, mood, pain appreciation, etc., and more specifically, alertness, depression and/or anxiety. For example, when administered into the ventricles, CRF increases activity and improves learning performance in rats and thus may function as a natural stimulant.

CRF analogs should also be of use for increasing blood flow to the gastrointestinal tract of mammals, particularly humans and other mammals. All CRF related peptides have been shown to dilate the mesenteric vascular bed. Also, oCRF inhibits gastric acid production, and CRF analogs are expected to also be effective in the treatment of gastric ulcers by reducing gastric acid production and/or inhibiting gastrointestinal functions in a mammal.

CRF analogs or the nontoxic addition salts thereof, combined with a pharmaceutically or veterinarily acceptable carrier to form a pharmaceutical composition, may be administered to mammals, including humans, either intravenously, subcutaneously, intramuscularly, percutaneously, e.g. intranasally, intracerebrospinally or orally. The peptides should be at least about 90% pure and preferably should have a purity of at least about 98%; however, lower purities are effective and may well be used with mammals other than humans. This purity means that the intended peptide constitutes the stated weight % of all like peptides and peptide fragments present. Administration to humans may be employed by a physician to lower blood pressure or to stimulate endogenous gluco-corticoid production. The required dosage will vary with the particular condition being treated, with the severity of the condition and with the duration of desired treatment.

These peptides may also be used to evaluate hypothalamic pituitary adrenal function in mammals with suspected endocrine or central nervous system pathology by suitable administration followed by monitoring body functions. For example, administration may be used as a diagnostic tool to evaluate Cushing's disease and affective disorders, such as depressive illness.

Such peptides are often administered in the form of pharmaceutically or veterinarily acceptable nontoxic salts, such as acid addition salts or metal complexes, e.g., with zinc, iron, calcium, barium, magnesium, aluminum or the like (which are considered as addition salts for purposes of this application). Illustrative of such acid addition salts are hydrochloride, hydrobromide, sulphate, phosphate, tannate, oxalate, fumarate, gluconate, alginate, maleate, acetate, citrate, benzoate, succinate, malate, ascorbate, tartrate and the like. If the active ingredient is to be administered in tablet form, the tablet may contain a binder, such as tragacanth, corn starch or gelatin; a disintegrating agent, such as alginic acid; and a lubricant, such as magnesium stearate. If administration in liquid form is desired, sweetening and/or flavoring may be used, and intravenous administration in isotonic saline, phosphate buffer solutions or the like may be effected.

The peptides should be administered under the guidance of a physician, and pharmaceutical compositions will usually contain the peptide in conjunction with a conventional, pharmaceutically or veterinarily-acceptable carrier. Usually, the dosage will be from about 1 to about 200 micrograms of the peptide per kilogram of the body weight of the host animal. In some instances, treatment of subjects with these peptides can be carried out in lieu of the administration of ACTH or corticosteroids, in such instances a dosage as low as about 10 ng/Kg of body weight may be employed. As used herein all temperatures are °C and all ratios are by volume. Percentages of liquid materials are also by volume.

Although the invention has been described with regard to its preferred embodiments, which constitute the best mode presently known to the inventors, it should be understood that various changes and modifications as would be obvious to one having the ordinary skill in this art may be made without departing from the scope of the invention which is set forth in the claims appended hereto. For example, substitutions and modifications at other positions in the CRF peptide chain can be made in accordance with present or future developments without detracting from the potency of the analogs. It appears important that the amino acid sequence from about positions 6 through 41 or an equivalent sequence of that length be present in the synthetic peptide, whereas the remainder of the N-terminus of the molecule does not appear as critical. In addition, instead of the simple amide at the C-terminus, a lower alkyl-substituted amide, e.g. methylamide, ethylamide, etc, may be incorporated, and such substituted amides are considered equivalent to the unsubstituted amide. Likewise from one to ten additional amino acid residues can be included at the N-terminus without significantly adversely affecting biological potency. Such peptides are considered as equivalents which fall within the scope of the claims

## Claims

1. A peptide having the formula: 
Y-R-R1-R2-
R3-R4-R5-Ile-Ser-R8-R9-leu-R11-R12-R13-R14-leu-Arg-R17-R18-
R19-His-R21-R22-R23-R24-R25-R26-R27-R28-R29-Gln-ala-R32-R33-
Asn-Arg-R36-R37-R38-R39-R40-R41-NH2
 wherein Y is an acyl group having 7 or less carbon atoms or hydrogen; R is a subsequence of from 1 to 10 amino acid residues or is des-R; R₁ is Ser, D-Ser or des R₁; R₂ is Glu, Gln, pGlu, D-pGlu or des R₂; R₃ is Glu, Gly, D-Tyr or des R₃; R₄ is Pro, D-Pro or des R₄; R₅ is Pro or desR₅; R₈ and R₁₉ are selected from the group consisting of leu, Ile, ala, Gly, Val, Nle, Phe and Gln; R₉ is Asp or Glu; R₁₁ is Thr or Ser; R₁₂ is Phe, leu, ala, Ile, Gly, Val, Nle or Gln; R₁₃ is His, Tyr or Glu; R₁₄ is leu or Met; R₁₇ is Glu or Lys; R₁₈ is Val, Nle or Met; R₂₁ is Arg, Met, Nva, Ile, ala, leu, Nle, Val, Phe or Gln; R₂₂ is ala, Thr, Asp or Glu; R₂₃ is Arg, Orn, Har or Lys; R₂₄ is Met, leu, Ile, ala, Gly, Val, Nle, Phe and Gln; R₂₅ is Glu or Asp; R₂₆ is Gly, Gln, Asn or Lys; R₂₇ is leu, Ile, ala, Val, Nva, Met, Nle, Phe, Asp, Asn, Gln or Glu; R₂₈ is ala, Arg or Lys; R₂₉ is Gln or Glu; R₃₂ is Leu, His, Gly, Tyr or ala; R₃₃ is Ile, Ser, Asn leu, Thr or ala; R₃₆ is Asn, Lys, Orn, Arg, Har or Leu; R₃₇ is leu or Tyr; R₃₈ is Met or leu; R₃₉ is Glu or Asp; R₄₀ is Ile, Thr, Glu, ala, Val, leu, Nle, Phe, Nva, Gly, Asn or Gln; and R₄₁ is Ile, ala, Gly, Val, Leu, Nle, Phe, Gln or des R₄₁.

2. A peptide as claimed in Claim 11 wherein R₃₆ is Asn.

3. A peptide as claimed in Claim 1 or Claim 2 wherein R₂₁ is Arg.

4. A peptide as claimed in any preceding claim wherein R₃₂ is Leu.

5. A peptide as claimed in any preceding claim wherein R₃₃ is Ile.

6. A peptide as claimed in any preceding claim wherein R₂₆ is Gly.

7. A peptide as claimed in any preceding claim wherein R₁₄ is Met.

8. A peptide as claimed in any preceding claim wherein R₂₄ is Met.

9. A peptide as claimed in Claim 1 and having the formula:

H-Ser-Glu-Glu-Pro-Pro-Ile-Ser-Leu-Asp-Leu-
Thr-Phe-His-Leu-Leu-Arg-Glu-Val-Leu-His-Met-Ala-Arg-Ala-
Glu-Gln-Leu-Ala-Gln-Gln-Ala-His-Ser-Asn-Arg-Lys-Leu-Met-
Glu-Ile-Ile-NH2;

10. A peptide as claimed in Claim 1 and having the formula:

H-Ser-Glu-Glu-Pro-Pro-Ile-Ser-Leu-Asp-Leu-
Thr-Phe-His-Leu-Leu-Arg-Glu-Val-Leu-His-Met-Ala-Arg-Ala-
Glu-Gly-Leu-Ala-Gln-Gln-Ala-His-Ser-Asn-Arg-Lys-Leu-Met-
Glu-Ile-Ile-NH2.

11. A peptide as claimed in Claim 1 and having the formula:

H-Ser-Gln-Glu-Pro-Pro-Ile-Ser-Leu-Asp-Leu-
Thr-Phe-His-Leu-Leu-Arg-Glu-Val-Leu-His-Met-Thr-Lys-Ala-
Asp-Gln-Leu-Ala-Gln-Gln-Ala-His-Ser-Asn-Arg-Asn-Leu-Leu-
Asp-Ile-Ala-NH2.

12. A non-toxic addition salt of a peptide as claimed in any preceding claim.

13. A composition for lowering the blood pressure of mammals and comprising an effective amount of a synthetic CRF peptide Claim 1 or a nontoxic addition salt thereof and a pharmaceutically or veterinarily acceptable liquid or solid carrier therefor.

## Patentansprüche

1. Peptid der Formel: 
Y-R-R1-R2-R3-R4-R5-Ile-Ser-R8-R9-leu-
R11-R12-R13-R14-leu-Arg-R17-R18-R19-His-R21-R22-R23-R24-R25-
R26-R27-R28-R29-Gln-ala-R32-R33-Asn-Arg-R36-R37-R38-R39-R40-
R41-NH2,
 worin Y eine Acylgruppe mit 7 oder weniger Kohlenstoffatomen oder Wasserstoff ist; R eine Subsequenz von 1 bis 10 Aminosäureresten oder des-R bedeutet; R₁ Ser, D-Ser oder des R₁ ist; R₂ Glu, Gln, pGlu, D-pGlu oder des R₂ ist; R₃ Glu, Gly, D-Tyr oder des R₃ ist; R₄ Pro, D-Pro oder des R₄ ist; R₅ Pro oder desR₅ ist; R₈ und R₁₉ aus der Gruppe bestehend aus leu, Ile, ala, Gly, Val, Nle, Phe und Gln gewählt sind; R₉ Asp oder Glu ist; R₁₁ Thr oder Ser ist; R₁₂ Phe, leu, ala, Ile, Gly, Val, Nle oder Gln ist; R₁₃ His, Tyr oder Glu ist; R₁₄ leu oder Met ist; R₁₇ Glu oder Lys ist; R₁₈ Val, Nle oder Met ist; R₂₁ Arg, Met, Nva, Ile, ala, leu Nle, Val, Phe oder Gln ist; R₂₂ ala, Thr, Asp oder Glu ist; R₂₃ Arg, Orn, Har oder Lys ist; R₂₄ Met, leu Ile, ala, Gly, Val, Nle, Phe und Gln ist; R₂₅ Glu oder Asp ist; R₂₆ Gly, Gln, Asn oder Lys ist; R₂₇ leu, Ile, ala, Val, Nva, Met, Nle, Phe, Asp, Asn, Gln oder Glu ist; R₂₈ ala, Arg oder Lys ist; R₂₉ Gln oder Glu ist; R₃₂ Leu, His, Gly, Tyr oder ala ist; R₃₃ Ile, Ser, Asn, leu, Thr oder ala ist; R₃₆ Asn, Lys, Orn, Arg, Har oder Leu ist; R₃₇ leu oder Tyr ist; R₃₈ Met oder leu ist; R₃₉ Glu oder Asp ist; R₄₀ Ile, Thr, Glu, ala, Val, leu, Nle, Phe, Nva, Gly, Asn oder Gln ist; und R₄₁ Ile, ala, Gly, Val, Leu, Nle, Phe, Gln oder des R₄₁ ist.

2. Peptid nach Anspruch 1, worin R₃₆ Asn ist.

3. Peptid nach Anspruch 1 oder 2, worin R₂₁ Arg ist.

4. Peptid nach einem der vorhergehenden Ansprüche, worin R₃₂ Leu ist.

5. Peptid nach einem der vorhergehenden Ansprüche, worin R₃₃ Ile ist.

6. Peptid nach einem der vorhergehenden Ansprüche, worin R₂₆ Gly ist.

7. Peptid nach einem der vorhergehenden Ansprüche, worin R₁₄ Met ist.

8. Peptid nach einem der vorhergehenden Ansprüche, worin R₂₄ Met ist.

9. Peptid nach Anspruch 1, welches die folgende Formel aufweist:

H-Ser-Glu-Glu-Pro-Pro-Ile-Ser-Leu-Asp-Leu-Thr-Phe-
His-Leu-Leu-Arg-Glu-Val-Leu-His-Met-Ala-Arg-Ala-Glu-Gln-Leu-
Ala-Gln-Gln-Ala-His-Ser-Asn-Arg-Lys-Leu-Met-Glu-Ile-Ile-NH2.

10. Peptid nach Anspruch 1, welches die folgende Formel aufweist:

H-Ser-Glu-Glu-Pro-Pro-Ile-Ser-Leu-Asp-Leu-Thr-Phe-
His-Leu-Leu-Arg-Glu-Val-Leu-His-Met-Ala-Arg-Ala-Glu-Gly-Leu-
Ala-Gln-Gln-Ala-His-Ser-Asn-Arg-Lys-Leu-Met-Glu-Ile-Ile-NH2.

11. Peptid nach Anspruch 1, welches die folgende Formel aufweist:

H-Ser-Gln-Glu-Pro-Pro-Ile-Ser-Leu-Asp-Leu-Thr-Phe-
His-Leu-Leu-Arg-Glu-Val-Leu-His-Met-Thr-Lys-Ala-Asp-Gln-Leu-
Ala-Gln-Gln-Ala-His-Ser-Asn-Arg-Asn-Leu-Leu-Asp-Ile-Ala-NH2.

12. Nicht-toxisches Additionssalz eines Peptides nach einem der vorhergehenden Ansprüche.

13. Zusammensetzung Zum Erniedrigen des Blutdruckes von Säugern, welche eine wirksame Menge eines synthetischen CRF-Peptides nach Anspruch 1 oder ein nicht-toxisches Additionssalz davon und einen pharmazeutisch oder veterinär verträglichen flüssigen oder festen Träger dafür enthält.

## Revendications

1. Peptide répondant à la formule : 
Y-R-R1-
R2-R3-R4-R5-Ile-Ser-R8-R9-leu-R11-R12-R13-R14-leu-Arg-R17-R18-R19-
His-R21-R22-R23-R24-R25-R26-R27-R28-R29-Gln-ala-R32-R33-Asn-Arg-R36-
R37-R38-R39-R40-R41-NH2
 dans laquelle Y est un groupe comportant 7 atomes de carbone ou moins ou de l'hydrogène, R est une sous-séquence de 1 à 10 restes d'acide aminé ou est des-R; R₁ est Ser, D-Ser ou des R₁; R₂ est Glu, Gln, pGlu, D-pGlu ou des R₂; R₃ est Glu, Gly, D-Tyr ou des R₃; R₄ est Pro, D-Pro ou des R₄; R₅ est Pro ou desR₅; R₈ et R₁₉ sont choisis dans le groupe comprenant leu, Ile, ala, Gly, Val, Nle, Phe et Gln; R₉ est Asp ou Glu; R₁₁ est Thr ou Ser; R₁₂ est Phe, leu, ala, Ile, Gly, Val, Nle ou Gln; R₁₃ est His, Tyr ou Glu; R₁₄ est leu ou Met; R₁₇ est Glu ou Lys; R₁₈ est Val, Nle ou Met; R₂₁ est Arg, Met, Nva, Ile, ala, leu, Nle, Val, Phe ou Gln, R₂₂ est ala, Thr, Asp ou Glu; R₂₃ est Arg, Orn, Har ou Lys; R₂₄ est Met, leu, Ile, ala, Gly, Val, Nle, Phe ou Gln; R₂₅ est Glu ou Asp; R₂₆ est Gly, Gln, Asn ou Lys; R₂₇ est leu, Ile, ala, Val, Nva, Met, Nle, Phe, Asp, Asn, Gln ou Glu; R₂₈ est ala, Arg ou Lys; R₂₉ est Gln ou Glu; R₃₂ est Leu, His, Gly, Tyr ou ala; R₃₃ est Ile, Ser, Asn leu, Thr ou ala; R₃₆ est Asn, Lys, Orn, Arg, Har ou Leu; R₃₇ est leu ou Tyr; R₃₈ est Met ou leu; R₃₉ est Glu ou Asp; R₄₀ est Ile, Thr, Glu, ala, Val, leu, Nle, Phe, Nva, Gly, Asn ou Gln; et R₄₁ est Ile, ala, Gly, Val, Leu, Nle, Phe, Gln ou des R₄₁.

2. Peptide suivant la revendication 1, dans lequel R₃₆ est Asn.

3. Peptide suivant l'une ou l'autre des revendications 1 et 2, dans lequel R₂₁ est Arg.

4. Peptide suivant l'une quelconque des revendications précédentes, dans lequel R₃₂ est Leu.

5. Peptide suivant l'une quelconque des revendications précédentes, dans lequel R₃₃ est Ile.

6. Peptide suivant l'une quelconque des revendications précédentes, dans lequel R₂₆ est Gly.

7. Peptide suivant l'une quelconque des revendications précédentes, dans lequel R₁₄ est Met.

8. Peptide suivant l'une quelconque des revendications précédentes, dans lequel R₂₄ est Met.

9. Peptide suivant la revendication 1 et répondant à la formule :

H-Ser-Glu-Glu-Pro-Pro-Ile-Ser-
Leu-Asp-Leu-Thr-Phe-His-Leu-Leu-Arg-Glu-Val-Leu-His-Met-
Ala-Arg-Ala-Glu-Gln-Leu-Ala-Gln-Gln-Ala-His-Ser-Asn-Arg-
Lys-Leu-Met-Glu-Ile-Ile-NH2.

10. Peptide suivant la revendication 1 et répondant à la formule :

H-Ser-Glu-Glu-Pro-Pro-Ile-Ser-
Leu-Asp-Leu-Thr-Phe-His-Leu-Leu-Arg-Glu-Val-Leu-His-Met-
Ala-Arg-Ala-Glu-Gly-Leu-Ala-Gln-Gln-Ala-His-Ser-Asn-Arg-
Lys-Leu-Met-Glu-Ile-Ile-NH2.

11. Peptide suivant la revendication 1 et répondant à la formule :

H-Ser-Gln-Glu-Pro-Pro-Ile-Ser-
Leu-Asp-Leu-Thr-Phe-His-Leu-Leu-Arg-Glu-Val-Leu-His-Met-
Thr-Lys-Ala-Asp-Gln-Leu-Ala-Gln-Gln-Ala-His-Ser-Asn-Arg-
Asn-Leu-Leu-Asp-Ile-Ala-NH2.

12. Sel d'addition non toxique d'un peptide suivant l'une quelconque des revendications précédentes.

13. Composition pour abaisser la pression sanguine des mammifères et comprenant une quantité efficace d'un peptide CRF synthétique suivant la revendication 1 ou d'un sel d'addition non toxique de celui-ci et un support liquide ou solide acceptable du point de vue pharmaceutique ou vétérinaire.
